# EUROPÄISCHE PATENTSCHRIFT

(11) **EP 4 173 598 B1**
(45) Veröffentlichungstag und Bekanntmachung des Hinweises auf die Patenterteilung: **08.01.2025**
(21) Anmeldenummer: 21204837.5
(22) Anmeldetag: 26.10.2021
(51) Int. Cl.: A61F 2/46, A61B 9/00

(54) **ERFINDUNG BETREFFEND HAMMERSCHLAGMONITORING**
HAMMER IMPACT MONITORING
SURVEILLANCE DES COUPS DE MARTEAU

(43) Veröffentlichungstag der Anmeldung: 03.05.2023
(73) Patentinhaber: Justus-Liebig-Universität Gießen, Körperschaft des öffentlichen Rechts, 35390 Gießen (DE); Technische Hochschule Mittelhessen, Körperschaft des öffentlichen Rechts, 35390 Giessen (DE)
(72) Erfinder: Fonseca-Ulloa, Carlos Alfonso, 35394 Giessen (DE); Schreynemackers, Simon, 61169 Friedberg (DE); Jahnke, Alexander, 35392 Gießen (DE); Ishaque, Bernd, 35394 Gießen (DE)
(74) Vertreter: Stumpf, Peter

(56) Entgegenhaltungen:
- EP-A1- 3 260 088
- EP-A1- 3 524 197
- WO-A2-2012/021894
- US-A1- 2017 112 634

## Beschreibung

Die vorliegende Erfindung betrifft ein Verfahren und Vorrichtung zum Hammerschlagmonitoring zur Überwachung von Implantationen von Prothesen in Knochen mittels eines Operationshammers.

### Beschreibung und Einleitung des allgemeinen Gebietes der Erfindung

Eine Implantation ist eine Operation zu dem Einsetzen eines künstlichen Materials in den Körper. Das eingesetzte Material welches auch als Implantat oder als Prothese bezeichnet wird verbleibt dabei permanent oder zumindest für einen längeren Zeitraum im Körper. Die Implantationen von Prothesen in Knochen erfolgt üblicherweise mittels eines Operationshammers. Dabei ist es von großer Bedeutung, dass dessen Schläge mit der richtigen Kraft ausgeführt werden. Operationshämmer werden i.d.R. für das Einschlagen von Marknägeln, Raspeln, Endoprothesen und das Fügen von Köpfen von Hüftendoprothesen verwendet. Es gibt allein in Deutschland (jährlich ca. 250.000 primäre Hüftimplantationen und 160.000 Knieimplantationen. Bis dato gibt es hierfür allerdings kein ausreichendes Messgerät um den richtigen Einsatz des Operationshammers (z.B. hinsichtlich der aufgewandten Kraft) zu überwachen.

### Stand der Technik

Es ist mit bestehenden Lösungen grundsätzlich möglich, die Schallemission eines Operationshammers während einer Operation zu messen.

Die Variabilität der patientenindividuellen Schallemissionen ist jedoch schwer zu normieren. Die Schrift US15/315,619 beschreibt eine Möglichkeit zur akustischen Überwachung des Implantationsprozesses. Diese stellt aber nur eine ortsbezogene temporäre Lösung dar. Die Implantation wird dabei nur als Ganzes akustisch bewertet und nicht die konkreten Einzelschritte bei der Implantation der Prothese.

Patent Dokument US 2017/112634 A1 offenbart ein Verfahren zum Hammerschlagmonitoring zur Überwachung von Implantationen von Prothesen in Knochen mittels eines Operationshammers wenigstens umfassend folgende Schritte: a) Erfassung der Schallemissionen in einem Operationssaal als Audio-Messdaten während einer Implantation; b) Vorverarbeitung und Segmentierung der Audio-Messdaten aus Schritt a); c) Ermittlung der Klassifikationsdaten wenigstens aus den vorverarbeiteten Audio-Messdaten aus Schritt b) mittels des trainierten Künstlichen Neuronalen Netzes (KNN), welche wenigstens einen Klassifikationswert für wenigstens einen Schlag eines Operationshammers auf eine Prothese umfassen,sodass aus diesen Klassifikationsdaten eine Bewertung der Schlagqualität erfolgen kann, d) Ausgabe der Klassifikationsdaten aus Schritt c) zur Überwachung von Implantationen von Prothesen.

### Aufgabe

Aufgabe der vorliegenden Erfindung ist es, die Nachteile des Standes der Technik zu vermeiden, sodass ein kontinuierliches Monitoring des Implantationsprozesses ermöglicht wird. Dabei wird der Einsatz eines Operationshammers insbesondere hinsichtlich der aufgewandten Kraft überwacht.

Dies dient zur Feststellung des ersten Momentes der Festigkeit zwischen der zu implantierenden Prothese und Knochen, um Frakturen und Schäden des Prothesenlagers durch übermäßigen Kraftaufwand am Markraum zu verhindern.

### Lösung der Aufgabe

Die Lösung dieser Aufgaben ergibt sich aus den Merkmalen des Hauptanspruchs. Weiterhin sind vorteilhafte Ausgestaltungen und Weiterbildungen der Erfindung den Unteransprüchen entnehmbar.

Zunächst erfolgt eine Definition, wie einige für das erfindungsgemäße Verfahren maßgebliche Begriffe im Rahmen dieser Erfindung zu verstehen sind:
Klinische Parameter:
Das Monitoring erfolgt durch eine Auswertung der Audio-Messwerte während der Operation. Dies kann durch Hinzunahme und Auswertung zusätzlicher klinischer Parameter verbessert werden. Diese Parameter sind beispielsweise Knochendichte (BMD), Größe, Gewicht, Alter, Canal Flare Index (CFI) und BMI des zu operierenden Patienten.

Eine höhere Knochendichte (BMD) ändert die Audio-Messwerte bei den ersten Schlägen des Operationshammers in bestimmten Frequenzbändern. Das bedeutet, dass eine höhere Knochendichte die Unterschiede der Audio-Messwerte zwischen Schläge beeinflusst. Damit sollte sie vorzugsweise als wichtige Parameter für die genauere Differenzierung der Audio-Messwerte in Abhängigkeit der Schlagkraft einbezogen werden.

Gewicht, Größe und BMI spielen als Dämpfungsfaktoren des Schalls und daher unterscheiden sich die Audio-Messwerte in Abhängigkeit von den entsprechenden Körpereigenschaften des Patienten. Das bedeutet für dieses System, dass diese Parameter, die vorzugsweise vor der Implantation erfasst werden, in den Algorithmus als unabhängige Gewichtungsfaktoren integriert werden, um das Zusammenhängen zwischen Festigkeit und Schlagklang zu verfeinern.

Der Canal Flare Index (CFI das Verhältnis des Durchmessers des Femurkanals am Isthmus in der anteroposterioren Ansicht zum Durchmesser des Markkanals 20 mm oberhalb des Trochanter minor) beeinflusst die Press-Passung der zu implantierenden Prothese bei Implantationen in den Oberschenkelknochen, damit ist dieser Parameter hiervon großer Bedeutung und das Audio-Messignal richtig bei der Bestimmung der Kraft zu berücksichtigen.

### Künstliche Neuronale Netze:

Künstliche Neuronale Netze, auch Künstliche Netzwerke, kurz: KNN (englisch artificial neural network, ANN), sind Netze aus künstlichen Neuronen. Künstliche Neuronale Netze basieren auf der Vernetzung vieler einzelner, meist in Schichten angeordneter, künstlicher Neuronen. Die Intensität der Verbindungen zwischen den Neuronen wird durch sogenannte Gewichtungen/Gewichte gesteuert. Die Topologie sowie die steuernden Parameter eines Netzes werden in Abhängigkeit der Problemstellung gewählt.

In einer Trainingsphase werden KNN anhand von Eingangsdaten (Eingangssignal), für die erwartete Ergebnisse (Soll-Signal) vorliegen, durch Vergleich dieser mit den errechneten Ergebnissen (Ergebnis-Signal) angelernt.

Die Differenz von Ergebnis- und Soll-Signal wird als Loss bezeichnet. Der Loss zeigt an, wie gut das erwartete und das tatsächliche Ergebnis übereinstimmen. Um die Qualität der Verarbeitung zu verbessern, muss dieser Loss für zukünftige Durchläufe berücksichtigt werden. Diese Anpassung der Verarbeitung bezeichnet man als Backpropagation bzw. Gewichts-Anpassung. Hierbei werden die Gewichte zwischen den Neuronen derart justiert, dass sie zukünftig dem gewünschten Ergebnis näherkommen.

Basierend darauf kann die Lernfähigkeit von KNNs als Fähigkeit zum Vergleich eines bestimmten Ergebnisses und eines erwarteten Ergebnisses, gefolgt von einer Anpassung entsprechend der Differenz zwischen diesen Werten, definiert werden.

KNNs sind dadurch in der Lage, mithilfe eines iterativen Trainingsprozesses komplizierte nichtlineare Funktionen zu erlernen.

### Erfindungsgemäßes Verfahren:

Die in einem vorangegangenen Schritt (der nicht Teil des erfindungsgemäßen Verfahrens ist) erhobenen Audio-Trainingsmessdaten TD1 und klinischen Parameter D2 werden als Eingangssignale des erfindungsgemäßen Verfahrens verwendet.

Die klinischen Parameter D2 werden - nach eventueller Imputation - in die Verarbeitung mit einbezogen. Imputation bedeutet, dass fehlende Daten für die Parameter D2 (unvollständige Datensätze) in statistischen Erhebungen - die sogenannten Antwortausfälle - in der Datenmatrix vervollständigt werden. Die Schweigeverzerrung, die durch die Antwortausfälle entsteht, wird dadurch verringert. Es gibt dabei verschiedene Verfahren, mit denen fehlende Werte vervollständigt werden, diese sind allgemein bekannt. Dabei unterscheidet man grob zwischen der singulären und der multiplen Imputation.

Für das Training des neuronalen Netzes werden bevorzugt wenigstens ein klinischer Parameter D2, ein Audioausschnitt TD1 verwendet. Die klinischen Parameter wie auch der Audio-Ausschnitt stellen dabei die Eingangssignale (TD1, D2) dar, während die Referenz D3 die Zielwerte für die Ausgangs-Signale des Netzes bereitstellt. Diese Menge von Tupeln aus (x, y) bilden die Trainingsdaten (Dtraining). Hierbei entspricht ein x einem Tupel aus den Daten TD1 und vorzugsweise D2 (Audio-Ausschnitt, klinische Parameter) und ein y einem Vektor mit der Referenzinformation D3. Beim Audio-Ausschnitt handelt es sich um eine Matrix aus #LeadsAudio und #SequenzlängeAudio, während die klinischen Parameter D2 in Form eines Vektors mit den Parametern als Einträge bereitgestellt werden.

Aus den Eingangssignalen x wird über das KNN ein Ergebnissignal y' ermittelt, was wie zuvor beschrieben mit dem wahren y abgeglichen und zur Berechnung des Loss genutzt wird.

### Trainingsphase

Vor der eigentlichen Durchführung des erfindungsgemäßen Verfahrens muss das KNN eine Trainingsphase als einen Schritt 0 durchlaufen.

Dieser Schritt 0 umfasst folgende Teilschritte:
**0a. Empfangen der Audio-Trainingsmessdaten TD1.**
**0b.** Vorverarbeitung der Audio-Trainingsmessdaten TD1 zu vorverarbeiteten Audio-Trainingsmessdaten TD1* und Übergabe dieser vorverarbeiteten Audio-Trainingsmessdaten TD1* an das Künstliche Neuronale Netz KNN.

### Vorverarbeitung in der Trainingsphase:

Um für das Training des Netzes qualitativ hochwertige Daten zu nutzen, müssen die verwendeten Daten wie bspw. Audio-Daten TD1 zu vorverarbeiten Audio-Daten TD1* aufbereitet werden.

Dazu muss eine sogenannte Daten-Pipeline eingerichtet werden. Diese definiert die einzelnen Teilschritte der Vorverarbeitung. Diese Daten-Pipeline dient dazu, die heterogen gespeicherten Daten in (von Störgeräuschen oder Artefakten) bereinigte Datensätze zu überführen, die problemlos im weiteren Verfahren zu verwenden sind.

Die Vorverarbeitung berücksichtigt geeignete Maßnahmen zur Anreicherung (Data Augmentation) der Daten.

Die Vorverarbeitung in der Trainingsphase umfasst folgende Schritte:
a. Extraktion

Die Audio-Daten der einzelnen Patienten werden aus den Rohdaten extrahiert. Das heißt aus der Aufzeichnung der Audio-Messwerte, werden Störgeräusche entfernt.

Die optionalen klinischen Parameter der einzelnen Patienten sowie die auf Basis des Referenzdaten getroffenen Klassifikationen werden aus den Patientenrohdaten-Dateien extrahiert. Diese liegen üblicherweise im CSV-Dateiformat vor. Anschließend werden die Audio-Daten, die klinischen Parameter und die Diagnose-Daten je einen Patienten zu einem Tupel (x, y) verknüpft.

### b. Auswahl

In diesem Schritt werden nicht verwendbare Datensätze von der weiteren Verarbeitung ausgeschlossen (z. B. unvollständige klinische Parameter, die nicht importiert werden können). Es sollen nur vollständige Datensätze für das Training genutzt werden, um das Erlernen von Korrelationen zu optimieren.

### c. Bereinigung

Die Audio-Aufzeichnungen werden beschnitten, um technische Artefakte z.B. am Beginn und/oder am Ende der Aufnahme zu beseitigen.

### d. Augmentierung

Um die für das Training verwendbare Datenmenge zu erhöhen und dadurch die Performance des Netzes zu steigern, findet eine Datenaugmentierung statt. Es empfiehlt sich eher mit Audioeffekten zu arbeiten die zuvor getestet keinen Einfluss auf das Klassifikationsergebnis aufweisen.

### e. Formatierung

Um hochfrequentes Rauschen und die Größe der Audio-Aufnahmen zu reduzieren, wird für jeden Datensatz ein Down-Sampling durchgeführt. Dies reduziert durch ein Average-Pooling-basiertes Verfahren Details der Aufnahme, erhält jedoch deren signifikante Muster. Hierdurch lässt sich die Datengröße um bspw. den Faktor 4 reduzieren.

### f. Speichern

Die Daten werden in gleichgroße Gruppen aufgeteilt. Abhängig vom gewünschten Verhältnis zwischen Trainingsdaten und Validierungsdaten (bevorzugt ist hierbei ein Verhältnis von 80% zu 20%) wird eine bestimmte Anzahl von Gruppen (z.B. 6) für das Training verwendet, der Rest für die Validierung. Diese Konstellation aus Trainings- und Validierungssplit wird je als eine Datensatz-Variante gespeichert. Dieser Vorgang wird so lange wiederholt, bis entweder genügend Varianten angelegt sind oder alle Kombinationen von Gruppen im Training/Validierung aufgetreten sind. Dies ermöglicht das Training identisch aufgebauter Modelle mit unterschiedlichen Daten. In Kombination liefern diese Modelle (z.B. 6) eine höhere Performance auf unbekannte Daten als ein einzelnes Modell aus dieser Menge. Man bezeichnet dieses Verfahren als Ensembling.

Anschließend erfolgen die weiteren Schritte:
**0c.** Ermittlung der Klassifikation für einen einzelnen Hammerschlag eines Operationshammers durch das Künstliche Neuronale Netz KNN.
**0d.** Eingabe der Referenz-Daten D3.

Die Referenzdaten D3 dienen zum Training des KNN.

**0e.** Iterativer Soll-Ist-Vergleich der ermittelten Wahrscheinlichkeiten mit den Referenz-Daten D3 mit anschließendem Anpassen der Gewichte G im Künstlichen Neuronalen Netz KNN.

**0f.** Fertigstellen des trainierten Künstlichen Neuronalen Netzes KNN aus den angepassten Gewichten G.

In einer Weiterbildung des Trainingsverfahrens erfolgt optional vor dem Schritt 0b noch ein zusätzlicher Schritt 0a* in welchem wenigstens ein klinischer Parameter D2 empfangen werden. In diesem Fall erfolgt anschließend ein zusätzlicher Schritt 0b*, welcher zwischen Schritt 0a* und Schritt 0c stattfindet. Schritt 0b* umfasst eine Vorverarbeitung des wenigstens einen klinischen Parameter D2 zu einem vorverarbeiten klinischen Parameter D2* und eine Übergabe dieses wenigstens einen vorverarbeiten klinischen Parameter D2* an das Künstliche Neuronale Netz KNN. Durch das Hinzuziehen der klinischen Parameter D2 kann die Zuverlässigkeit der Trainingsphase erhöht werden.

Nach der Trainingsphase wird das erfindungsgemäße Verfahren durchgeführt.

Das Verfahren zum Hammerschlagmonitoring zur Überwachung von Implantationen von Prothesen in Knochen mittels eines Operationshammers umfassend wenigstens folgende Schritte
a) Erfassung der Schallemissionen in einem Operationssaal als Audio-Messdaten D1 während einer Implantation und Übergabe der Audio-Messdaten D1 an ein trainiertes Künstliches Neuronales Netz (KNN)
   Hierbei werden die Audio-Messwerte im Operationsraum vorzugsweise kontinuierlich während des gesamten Operationsprozesses erfasst. Die Messung erfolgt dabei vorzugsweise durch wenigstens ein im Operationsraum angeordnetes Mikrofon. Die Messung erfolgt dabei außerhalb des Patienten. Das Ergebnis sind Audiodaten D1.
b) Vorverarbeitung und Segmentierung der Audio-Messdaten D1 aus Schritt a) durch das trainierte Künstliche Neuronale Netz (KNN), so-dass die durch Hammerschläge eines Operationshammers auf eine Prothese erzeugten Audio-Messdaten extrahiert und vorverarbeiteten Audio-Messdaten D1* erzeugt werden.
   Anschließend erfolgt eine Segmentierung und Filterung der in Schritt a übermittelten Audiodaten D1. Segmentierung heißt hierbei, dass die einzelnen Abschnitte der Audio-Messdaten D1 einzelnen Schläge des Operations-hammers auf eine zu implantierende Prothese zugeordnet werden. Die Segmentierung und Ausreißer-Detektion der einzelnen Schläge erfolgt dabei durch die erlernten Eigenschaften bzw. Filter der ersten Schichten des trainierten Künstlichen Neuronalen Netzes KNN. Das Ergebnis sind vorverarbeitete Audiodaten D1*.
c) Ermittlung der Klassifikationsdaten E wenigstens aus den vorverarbeiteten Audio-Messdaten D1* aus Schritt b) mittels des trainierten Künstlichen Neuronalen Netzes KNN, welche wenigstens einen Klassifikationswert für wenigstens einen Schlag eines Operations-hammers auf eine Prothese umfassen, sodass aus diesen Klassifikationsdaten E eine Bewertung der Schlagqualität erfolgen kann, wobei die vorverarbeiteten Audio-Messdaten D1* über den gesamten Verlauf einer Operation verwendet werden

Anschließend werden die vorverarbeiteten Audio-Daten D1* mittels des trainierten Künstlichen Neuronalen im Kontext der gesamten Implantation ausgewertet und aus den vorverarbeiteten Audio-Daten D1* Klassifikationsdaten E. berechnet. Diese Klassifikationsdaten E umfassen wenigstens einen Klassifikationswert für wenigstens einen Schlag eines Operationshammers auf eine zu implantierenden Prothese, sodass aus diesen Klassifikationsdaten E eine Einschätzung der Schlagqualität erfolgen kann, wobei die vorverarbeiteten Audiodaten D1 * über den gesamten Verlauf einer Operation verwendet werden.

Diese Daten sagen also aus, wie und ob der jeweilige Hammerschlag des Operationshammers korrekt durchgeführt wurde. Korrekt heißt hierbei, dass die von dem Operationshammer auf die zu implantierenden Prothese wirkende Kraft ausreichend groß war um diese sicher im Knochen zu verankern und nicht zu groß, so dass der Knochen geschädigt wird. Diese Werte sind aus der der medizinischen Praxis bekannt. Dabei wird jedes neue Paket der eingehenden Audiodaten D1 der zu analysierenden Sequenz hinzugefügt und durch bidirektionale rekursive Schichten im Zusammenhang der ganzen Messung betrachtet.

Die Audio-Messdaten D1 haben sich beispielsweise im Falle einer Veränderung des Knochen-Prothesenverbundes z.B. in Richtung einer erfolgreichen Implantation verändert. Das Netz erfasst den Gradienten dieser Veränderung und ermöglicht damit eine Prognose der noch zu applizierenden Kräfte.

### d) Ausgabe der Klassifikationsdaten E zur Überwachung von Implantationen von Prothesen

Hierbei werden die in Schritt d erzeugten Klassifikationsdaten E ausgeben. Dies dient dazu dem Operateur während der Implantation ein Feedback zu geben, ob die Operation korrekt verläuft oder die zu implantierenden Prothese nur zu lose beziehungsweise zu fest im Knochen verankert wird.

In einer alternativen Ausführungsform des Verfahrens umfasst weiterhin einen Schritt a1), der vor Schritt c) erfolgt. In diesem Schritt a1) wird wenigstens ein klinischer Parameter (D2) empfangen und an das trainierte Künstliches Neuronales Netz (KNN) übergeben, sodass in Schritt c) die Ermittlung der Klassifikationsdaten (E), wenigstens aus den vorverarbeiteten Audio-Messdaten (D1*) und dem wenigstens einem klinischen Parameters (D2) mittels des trainierten Künstlichen Neuronalen Netzes (KNN) erfolgt. Dabei umfassen die Klassifikationsdaten (E) wenigstens einen Klassifikationswert für wenigstens einen Schlag eines Operationshammers auf eine Prothese.

Durch das Hinzuziehen wenigstens eines klinischen Parameters D2 kann die Zuverlässigkeit des Verfahrens erhöht werden. Jeder Parameter der vorzugsweise vor die Implantation erfasst wird, wird im Algorithmus als unabhängiger Gewichtungsfaktor eingesetzt, um das Zusammenhängen zwischen Festigkeit und Schlagklang zu verfeinern.

Nachdem die Implantation abgeschlossen ist, werden entsprechend auffällige Schlagsequenzen analysiert und dem Operateur zur Beurteilung dargeboten. Zum Abschluss wird ein Bericht über die analysierte Implantation erstellt.

Das Verfahren dient zum kontinuierlichen Monitoring des Implantationsprozesses, zur Überwachung der Kraft der Schläge des Operationshammers zum Erreichen einer bestmöglichen Verbindung zwischen der zu implantierenden Prothese und dem Knochen, um Frakturen und Schäden des Prothesenlagers der u implantierenden Prothese durch übermäßigen Kraftaufwand am Markraum des Knochens zu verhindern. Ein gezieltes und intelligentes, optisches und akustisches Feedback soll den Operateur dabei unterstützen, den Prozess referenziert und dokumentiert durchzuführen. Darüber hinaus werden die gewonnenen und vom erfahrenen Operateur validierten Daten über eine Clientanwendung zum kontinuierlichen Training eines Künstlichen Neuronalen Netzes KNN verwendet, welches dezentralisiert auf einem Server mit multizentrischen Datensätzen gepflegt werden soll. Ein Update auf den Geräten des Operateurs erlaubt ihm ein evidenzbasiertes Implantationsmonitoring. Die Erfindung umfasst eine multizentrische Webanwendung, welche mit Hilfe von lokalen, im OP gewonnenen akustischen Daten, das zentrale Künstliche Neuronale Netz KNN verbessert und die Kommunikation und Dokumentation aller Implantationen, unabhängig von Standort, für alle Operateure erleichtert. Durch einen höheren telemedizinischen Austausch der Experten und dem automatisierten Überwachungssystem mittels eines Künstlichen Neuronalen Netzes wird die Qualitätssicherung einer Implantation verbessert.

Die Erweiterung dieses Ansatzes ermöglicht es durch die multizentrische Datenaufnahme und einen dezentralisierten Standort des Künstlichen Neuronalen Netzes KNN auf einem Server, die Probleme des lokalen und systematischen Trainingsbias der Festigkeitserkennung des Implantationsprozesses zu beheben.

Darüber hinaus ist mit diesem System eine Qualitätssicherung der Operation möglich, welche auch noch durch einen Post-hoc-Test analysiert werden kann. Post-hoc-Tests sind Signifikanztests aus der mathematischen Statistik. Mit der einfachen Varianzanalyse, dem Kruskal-Wallis-Test oder dem Median-Test wird nur festgestellt, dass es in einer Gruppe von Mittelwerten signifikante Unterschiede gibt. Damit können verschiedene Operationen miteinander verglichen werden und nicht nur das Ergebnis dokumentieren kann, sondern das vollständige Implantationsvorgehen speichert.

### Vorrichtung:

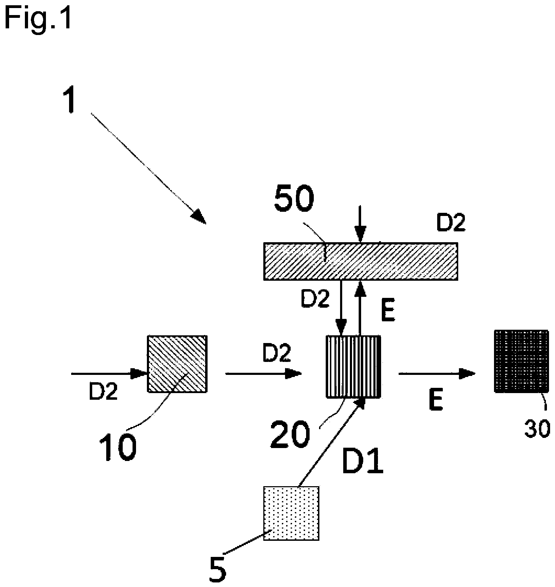

Die erfindungsgemäße Vorrichtung 1 zur Durchführung des Verfahrens zum Operationsmonitoring auf Basis von Audio-Aufzeichnungen und optional weiterer klinischer Parameter umfasst wenigstens die folgenden Elemente (siehe Abbildung 1):

### Ein Mikrofon 5

Das Mikrofon 5 ist so ausgebildet, dass es die während der Operation auftretenden Schallemissionen, insbesondere die Schallemissionen, welche bei der Implantation einer Prothese auftreten, aufnehmen und die resultierenden Audio-Messdaten D1 an eine Verarbeitungseinheit zur weiteren Verarbeitung übertragen kann. Der Frequenzbereich des Mikrofons 5 liegt dabei vorzugsweise bei 10 bis 50000 Hz. Die Datenübermittlung kann dabei über eine feste Datenleitung (z.B. über eine interne Datenleitung, Local-Area-Network (LAN) etc.), oder drahtlos (z.B. über Wireless Local-Area-Network (WLAN), Bluetooth etc.) erfolgen.

### Eine Eingabeeinheit 10:

Die Eingabeeinheit 10 ist so ausgebildet, dass über diese die Daten der klinischen Parameter D2 einzugeben sind und an die Auswerteeinheit 20 zur weiteren Verarbeitung zu übertragen sind. Bei der Eingabeeinheit 10 kann es sich um eine Tastatur oder um eine grafische Benutzeroberfläche (engl. GUI von graphical user interface) handeln. Die Datenübermittlung kann dabei über eine feste Datenleitung (z.B. über eine interne Datenleitung, Local-Area-Network (LAN) oder drahtlos (z.B. über Wireless Local-Area-Network (WLAN), Bluetooth) erfolgen. Dieses Baudelement ist optional.

### Eine Datenschnittstelle 50:

Die Datenschnittstelle 50 ist so ausgebildet, dass sie die Daten der klinischen Parameter D2 aus einem externen Datenverarbeitungssystem empfangen und die Klassifikationsdaten an ein externes Datenverarbeitungssystem weiterleiten kann. Die Datenübermittlung kann dabei über eine feste Datenleitung (z.B. über ein LAN etc.), oder drahtlos (z.B. über WLAN, Bluetooth etc.) erfolgen. Dieses Baudelement ist optional.

Weiterhin ist die Datenschnittstelle 50 so ausgebildet, dass sie Audio-Messdaten D1 und/oder vorzugsweise die Daten der klinischen Parameter D2 an die Auswerteinheit 20 übermitteln kann. Die Datenübermittlung kann dabei über eine feste Datenleitung (z.B. über ein LAN), oder drahtlos (z.B. über WLAN, Bluetooth) erfolgen. Ebenso kann die Übermittlung der Klassifikationsdaten E an eine externe Datenverarbeitungseinheit über die Datenschnittstelle 50 erfolgen.

Die Datenschnittstelle 50 ist üblicherweise auf den Austausch mit verschiedenen externen Datenverarbeitungssystemen ausgelegt, d.h. gängige Schnittstellen zum Datenaustausch sind bspw. USB, Cardreader, LAN, WLAN, Bluetooth.

Die Datenschnittstelle 50 ist dabei so ausgebildet, dass ein Anwender mit einem beliebigen webfähigen Gerät (bspw. Tablet, PC, Smartphone) im lokalen Netzwerk über die Datenschnittstelle 50 auf die Vorrichtung 1 zugreifen kann.

### Eine Auswertungseinheit 20:

Die Auswertungseinheit 20 ist so ausgebildet, dass sie die Audio-Messdaten D1 und die Daten der klinischen Parameter D2 von der Eingabeeinheit 10 oder einer Datenschnittstelle 50 empfangen kann. Die Datenübermittlung kann dabei über eine feste Datenleitung (z.B. über eine interne Datenleitung, LAN etc.), oder drahtlos (z.B. über WLAN, Bluetooth etc.).

Die Auswertungseinheit 20 ist weiterhin so ausgebildet, dass sie aus den Audio-Messdaten D1 und den klinischen Parametern D2 und aus den mittels Trainingsdaten enthaltenen erlernten Gewichten die Stärke und die räumliche Lokalisierung einer jedes Schlages des Operationshammers durch das künstliche Neuronale Netz berechnen kann. Das Ergebnis dieser Berechnung sind dabei die Klassifikationsdaten E. Als Auswertungseinheit 20 dient dabei ein Gerät zur elektronischen Datenverarbeitung, z.B. ein in die Vorrichtung 1 integrierter programmierbarer Mikroprozessor.

Die Auswertungseinheit 20 ist weiterhin so ausgebildet, dass sie die Klassifikationsdaten E an eine Ausgabeeinheit 30 übermitteln kann. Die Datenübermittlung kann dabei über eine feste Datenleitung (z.B. über eine interne Datenleitung, LAN etc.), oder drahtlos (z.B. über WLAN, Bluetooth etc.) erfolgen.

Die Auswertungseinheit 20 verwendet ein Künstliches Neuronales Netzwerk *KNN* zur Mustererkennung. Die Vorrichtung 1 berechnet Klassifikationsdaten E. Diese Daten sagen aus, wie und ob der jeweilige Hammerschlag des Operationshammers korrekt durchgeführt wurde.

iDiese Klassifikationsdaten E werden über die Ausgabeeinheit 30 in Form visueller und/oder akustischer Ausgaben ausgegeben. Alternativ werden sie an die Datenschnittstelle 50 weitergeleitet.

### Eine Ausgabeeinheit 30:

Die Ausgabeeinheit 30 ist so ausgebildet, dass sie die Klassifikationsdaten E der Auswertungseinheit 20 empfangen und dann ausgeben kann. Die Ausgabe kann optisch, akustisch, haptisch und/oder in Form eines Ausdruckes erfolgen.

Die Eingabeeinheit 10 bzw. die Ausgabeeinheit 30 der Vorrichtung 1 können dabei bspw. Tasten, LEDs, (Touch) Display und Sprachein-/ausgabe umfassen.

In einer ersten alternativen Ausführungsform ist die Vorrichtung 1 so aufgebaut, dass eine komplett gekapselte Verarbeitung der Daten unter Ausschluss jeglicher Netzzugriffe (LAN, WLAN) erfolgen kann. Dazu sind sämtliche Netzwerkkomponenten für jeden Datenaustausch der nicht zwischen Eingabeeinheit 10, Auswertungseinheit 20 und Ausgabeeinheit 30 erfolgt, gesperrt.

Mit Krankheitserregern kontaminierte Medizinprodukte (z.B. Messgeräte) können die Quelle von Infektionen beim Menschen sein. Die Anwendung solcher Medizinprodukte setzt daher eine vorhergehende Aufbereitung voraus, an die definierte Anforderungen zu stellen sind.

In einer zweiten alternativen Ausführungsform umfasst die Vorrichtung 1 deshalb ein Gehäuse, um die Auswertungseinheit 20, welches abwaschbar ausgebildet ist. Hierbei sind zusätzlich noch die Eingabeeinheit 10 und die Ausgabeeinheit 30 abwaschbar ausgebildet. Dazu weisen das Gehäuse 10, die Eingabeeinheit 10 und die Ausgabeeinheit 30 vorzugsweise eine Beschichtung aus Parylen oder aus wasserdichtem Silikon auf. Das ermöglicht eine besonders hygienische Verwendung.

(Ab-)Waschbar heißt hierbei: Die Oberfläche ist komplett geschlossen und lässt sich gemäß den Richtlinien des Verbunds für Angewandte Hygiene (VAH) oder der *Deutsche Gesellschaft für Hygiene und Mikrobiologie* (*DGHM*) mit allen gängigen Desinfektionsmitteln aufbereiten.

Vorzugsweise sind dabei das Gehäuse die Eingabeeinheit 10 und die Ausgabeeinheit 30 sterilisierbar nach DIN EN 285. Gemäß DIN EN 285 müssen alle Oberflächen der sterilisierten Gegenstände einem reinen, gesättigten Wasserdampf mit einer Temperatur von 134 °C über mindestens drei Minuten ausgesetzt werden. Ein dafür geeignetes Oberflächenmaterial ist beispielsweise PEEK (Polyetheretherketon).

Ein Aspekt der Nutzung eines dedizierten Gerätes ist der des Datenschutzes, da hier eine komplett gekapselte Verarbeitung der Daten unter Ausschluss jeglicher Netzzugriffe (LAN, WLAN) erfolgen kann, wenn dies erforderlich ist. Dadurch wird der Schutz sensibler Patientendaten gewährleistet. Das Gerät soll weiterhin die Sicherstellung von Hygiene und einer einfachen Einsetzbarkeit/Handhabbarkeit im klinischen Alltag unterstützen. Nicht überall kann ein PC mit Tastatur, bzw. ein Touchbasiertes Gerät (Tablet, Smartphone) eingesetzt werden. Dies hat sowohl hygienische als auch praktische Gründe. Ein für die Erkennung einer bestimmten Erkrankung dediziertes Gerät bietet den Vorteil der Kompaktheit, Bedienerfreundlichkeit, Hygiene und des Datenschutzes, da nur die für den Anwendungsfall notwendigen Ein- und Ausgabeeinheiten vorhanden sind. Ein sicheres, einfach zu reinigendes und bedienendes Gerät bietet daher wichtige und notwendige Aspekte eines Medizingerätes, weshalb ein solches auch Teil der Erfindung ist.

Das erfindungsgemäße Verfahren ist grundsätzlich für verschiedene Arten von Implantation geeignet bei denen Prothesen in Knochen mittels eines Operationshammers befestigt werden. Beispielhaft seien hier Operationen zum Ersetzen eines Hüftgelenks, eines Kniegelenks oder eines Schultergelenks genannt. Das Verfahren ist aber nicht darauf einschränkt. Dabei können sowohl das Implantat als auch der Operationshammer aus verschiedensten Materialen oder Materialkombinationen bestehen. Beispielshafte Gruppen von Materialien sind Edelstähle, Titanverbindungen, Keramiken oder Kunststoffe. Auch hier sind anderen Materialen möglich. Dazu ist es notwendig das Künstliche Neuronale Netz N auf die konkrete Art der Implantation und die Materialkombination des Operationshammers und der zu implantierenden Prothese zu trainieren.

### Ausführungsbeispiele

### Vor-Versuch 1:

Um Aussagen über die Eignung der Messmethode und deren Realisierbarkeit im Rahmen einer Operation zu gewinnen, wurden Testmessungen im Labor durchgeführt. Ziel dieses Vorversuchs war neben ausführlichen Tests der Sensorik das Generieren erster Datensätze und die Reliabilität des Auswertungsalgorithmus abzuschätzen. Für diese Messungen wurden 15 Implantationen einer Kurzschaftendoprothese in einen Kunstknochen von drei unterschiedlich erfahrenen Operateuren durchgeführt.

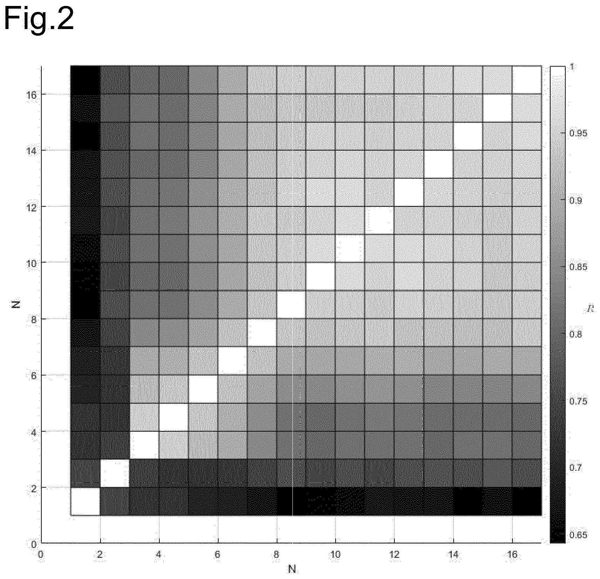

Die ersten Testmessungen dieses Implantationsprozesses weisen bei den verschiedenen Operateuren unterschiedliche Amplituden in der Systemantwort auf. Über den Verlauf der Testimplantation lassen sich dennoch Korrelationen zwischen der einzelnen energienormierten Systemantworten erkennen. Diese Systeme setzen sich aus verschiedenen schwingungsfähigen Komponenten (Hammer, Prothese, Implantationsbesteck) zusammen. Die daraus gewonnenen Schallsignale werden jeweils innerhalb einer Implantation korreliert. Die Abbildung 2 zeigt diese Korrelation. Gezeigt ist ein Trend in der Korrelation zwischen dem ersten Schlag N=1 und letzten Schlag N=14. Darüber hinaus konnte bei allen Messungen in der Korrelationsmatrix ein Trend zwischen dem Anfangsbereich der Schläge und dem Endbereich festgestellt werden. In einer weiteren Analyse wurden zur möglichen Falsifikation der Hypothese alle Implantationsschläge der drei Operateure in zufälliger Reihenfolge miteinander korreliert. Dabei ließ sich kein systematischer Trend in der Korrelationsmatrix erkennen. Das zeigt, dass der Trend der zeitabhängigen aufeinanderfolgenden Implantationsschläge nicht zufällig entstanden ist. Mit diesen Korrelationen ist es möglich, eine Ähnlichkeit der Schläge festzustellen.

Dieser Vorversuch hat die Zusammenhänge zwischen die Festigkeit und den Audio-Messdaten der Implantation in vitro dargestellt. Hierfür kann eine bestimmte Kraft zu einem bestimmten Schlagtonus darstellen. Darüber hinaus ist zu beobachten, wann die Prothese eine mechanische Festigkeit bekommen hat. Diese Festigkeit, auch Primärstabilität genannt, wurde über allgemein bekannte Primärstabilitätsanalysen geprüft und dargestellt.

### Vor-Versuch 2: In vivo

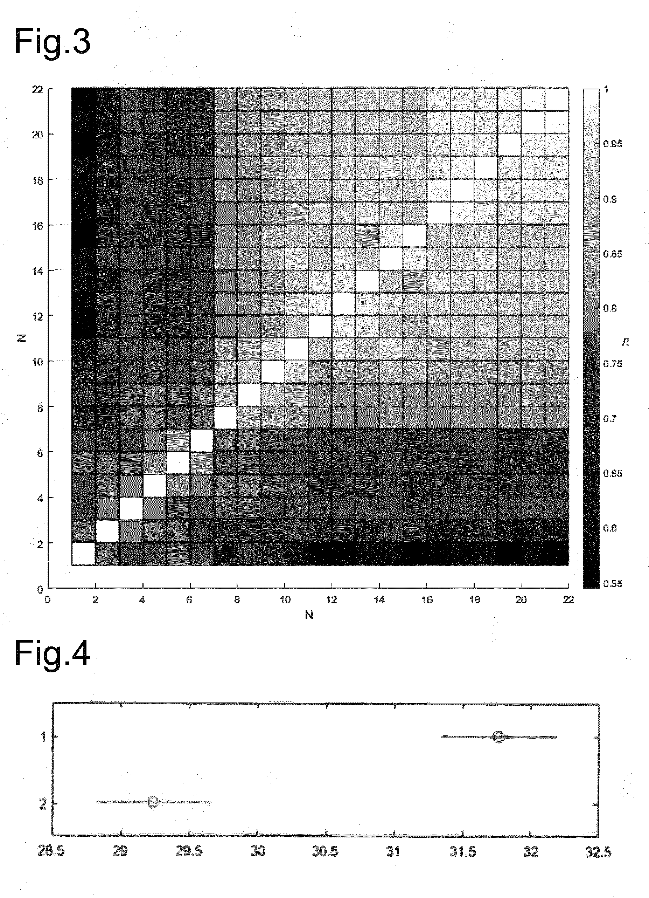

Für die Aufzeichnung der Audio-Messdaten wurde das Mikrofon der Vorrichtung 1 im Operationssaal platziert und die Erfassung der Schallemissionen als Audio-Messdaten gestartet. Nach dem Start der Aufnahme begann der Operateur die Schaftkomponente einzuschlagen, bis diese nach seinem Empfinden stabil verankert war. Dann wurde die Aufnahme gestoppt. Bei Betrachtung der Daten im energienormierten Powerspektrum fiel auf, dass sich bei allen Patienten, im Frequenzbereich zwischen 15000-24000Hz, keine Veränderungen während des Insertionsprozesses zeigten. Die mithilfe einer Diskrete Fourier-Transformation (DFT) generieten Powerspektren haben gezeigt, dass tendenziell mit steigender Anzahl der Hammerschläge, ein Wandel im Frequenzspektrum von dem oberen Frequenzbereich, hin zum unteren Frequenzbereich stattfindet. Diese Entwicklung konnte bei allen 30 Patienten festgestellt werden. Es wurde deutlich, dass bei den meisten Patienten der Korrelationskoeffizient von Schlag 1 zum finalen Schlag fast stetig anwuchs. Daher wurde, sowie bei dem ersten Versuch, ein tendenziell mit fortschreiten des Insertionsprozess wachsender Korrelationskoeffizient festgestellt (Abbildung Fig.3). Diese Sequenzen wurden dann in zwei Gruppen (Bereich 1 und Bereich 2) geteilt und anschließend automatisiert anhand der Korrelationsmatrix segmentiert. Die Einteilung in Bereich 1 und Bereich 2 dient dazu, die mittleren Amplitudenverläufe der Frequenzspektren gegeneinander aufzutragen. Die Unterschiede zwischen Bereich 1, welche die primären Schläge des Insertionsprozess abbildet, und Bereich 2, welche die finalen Schläge des Insertionsprozess abbildet, sind signifikant (Abbildung Fig.4)

Das vortrainierte Künstliche Neuronale Netz KNN bestehend aus einer rekurrenten Schrittfunktion (z.B. in der Form eines CNN's inkl. LSTM) und einer Überwachungsarchitektur ist zu Beginn einer Implantation mit den Patientendaten x₀ vorkonfiguriert worden.

CNN heißt dabei Convolutional Neural Network ist ein unterform eins künstlichen neuronalen Netzes. Es handelt sich um ein von biologischen Prozessen inspiriertes Konzept im Bereich des maschinellen Lernens. Convolutional Neural Networks finden Anwendung in zahlreichen Technologien der künstlichen Intelligenz, vornehmlich bei der maschinellen Verarbeitung von Bild- oder Audiodaten.

LSTM heißt dabei Long short-term memory (LSTM, deutsch: langes Kurzzeitgedächtnis) und ist eine Technik, die zur Verbesserung der Entwicklung von künstlichen Neuronalen Netzwerken.

Jeder Abschnitt Aₙ der n Implantationsschritte wird eigenständig vom Künstliche Neuronale Netz KNN analysiert. Die Ergebnisse En jedes Abschnittes An werden den nachfolgenden Abschnitten Aₙ₊₁ übergeben. Während eines An treten durch die Hammerschläge Schallemissionen xₙᵢ durch die Kraftapplikation auf den Prothesen-Knochenverbund auf. Jeder xₙᵢ wird segmentiert und an das vortrainierte Künstliche Neuronale Netz KNN zur Analyse weitergegeben. Das Künstliche Neuronale Netz KNN mit seiner rekurrenten Netzarchitektur kann aus xₙᵢ, allen vorherigen xₙ eines An und dem Eₙ₋₁ eine Ausgabe y'ₙᵢ erzeugen. Die Ausgabe y'ₙᵢ kann z.B. eine zu applizierende Kraft oder ein prozentualer Fortschritt der Implantation sein. Zusätzlich zu y'ₙ wird in jedem Schritt der Anwendung eine Übergangswahrscheinlichkeit λₙᵢ zum Beenden des Abschnittes An mit ausgegeben. Der Operateur hat somit die Möglichkeit während jedes Abschnittes der Operation einen Vorschlag zur Unterbrechung des einzelnen Abschnittes zu erhalten unter Betrachtung der prognostizierten Ausgaben y'ₙ welche somit auch im Verlauf abbildbar sind.

### Abbildungslegenden und Bezugszeichenliste

Fig. 1 zeigt eine schematische Darstellung der Vorrichtung 1. Die Eingabeeinheit 10 und die Datenschnittstelle 50 sind dabei optional.
Fig. 2 zeigt eine Korrelationsmatrix eines Implantationsvorgangs von Vor-Versuch 1.
Fig. 3 zeigt die Schlag-(N)-Sequenz-Korrelationsmatrix von Vor-Versuch 2.
Fig.4 zeigt den paarweisen Vergleich zwischen Bereich 1 und 2 für alle Patienten im Versuch 2.

### Bezugszeichen

- **1**: Vorrichtung
- **5**: Mikrofon
- **10**: Eingabeeinheit
- **20**: Auswertungseinheit
- **30**: Ausgabeeinheit
- **50**: Datenschnittstelle
- **TD1**: Audio-Trainingsmessdaten
- **TD1***: vorverarbeitete Audio-Trainingsmessdaten
- **D1**: Audio-Messdaten
- **D1***: vorverarbeitete Audio-Messdaten
- **D2**: klinische(r) Parameter
- **D3**: Referenz-Daten
- **E**: Klassifikationsdaten
- **KNN**: trainiertes Neuronales Netz
- **N**: Schlaganzahl
- **R**: Korrelationskoeffizient

## Patentansprüche

1. Verfahren zum Hammerschlagmonitoring zur Überwachung von Implantationen von Prothesen in Knochen mittels eines Operationshammers wenigstens umfassend folgende Schritte
a) Erfassung der Schallemissionen in einem Operationssaal als Audio-Messdaten (D1) während einer Implantation und Übergabe der Audio-Messdaten (D1) an ein trainiertes Künstliches Neuronales Netz (KNN)
b) Vorverarbeitung und Segmentierung der Audio-Messdaten (D1) aus Schritt a) durch das trainierte Künstliche Neuronale Netz (KNN), sodass die durch Hammerschläge eines Operationshammers auf eine Prothese erzeugten Audio-Messdaten extrahiert und vorverarbeiteten Audio-Messdaten (D1*) erzeugt werden.
c) Ermittlung der Klassifikationsdaten (E) wenigstens aus den vorverarbeiteten Audio-Messdaten (D1*) aus Schritt b) mittels des trainierten Künstlichen Neuronalen Netzes (KNN), welche wenigstens einen Klassifikationswert für wenigstens einen Schlag eines Operationshammers auf eine Prothese umfassen,
sodass aus diesen Klassifikationsdaten (E) eine Bewertung der Schlagqualität erfolgen kann, wobei die vorverarbeiteten Audio-Messdaten (D1*) über den gesamten Verlauf einer Operation verwendet werden
d) Ausgabe der Klassifikationsdaten (E) aus Schritt c) zur Überwachung von Implantationen von Prothesen

2. Verfahren nach Anspruch 1, **dadurch gekennzeichnet, dass** dieses weiterhin einen Schritt a1), der vor Schritt c) erfolgt, umfasst, in welchem wenigstens ein klinischer Parameter (D2) empfangen und an das trainierte Künstliches Neuronales Netz (KNN) übergeben wird, sodass in Schritt c) die Ermittlung der Klassifikationsdaten (E), wenigstens aus den vorverarbeiteten Audio-Messdaten (D1*) und dem wenigstens einem klinischen Parameters (D2) mittels des trainierten Künstlichen Neuronalen Netzes (KNN) erfolgt, wobei die Klassifikationsdaten (E) wenigstens einen Klassifikationswert für wenigstens einen Schlag eines Operationshammers auf eine Prothese umfassen

3. Vorrichtung zum Hammerschlagmonitoring zur Durchführung eines Verfahrens gemäß Anspruch 1 **dadurch gekennzeichnet, dass** diese wenigstens folgende Elemente umfasst:
• Mikrofon (5) welches so ausgebildet ist, dass es Audio-Messdaten (D1) aus Schallemissionen erzeugen und an eine Auswertungseinheit (20) weiterleiten kann,
• eine Auswertungseinheit (20) mit einem trainierten Künstlichen Neuronalen Netz (KNN), welche so ausgebildet ist, dass sie die Audio-Messdaten (D1) vom Mikrofon (5) empfangen, die Audio-Messdaten (D1) vorverarbeiten und segmentieren und so vorverarbeitete Audio-Messdaten (D1 *) erzeugen kann und weiterhin so ausgebildet ist, dass sie mittels des trainierten Künstlichen Neuronalen Netzes (KNN) aus den vorverarbeiteten Audio-Messdaten (D1*) Klassifikationsdaten (E) für wenigstens einen Schlag eines Operationshammers auf eine zu implantierenden Prothese durch das künstliche Neuronale Netz ermitteln kann und diese Klassifikationsdaten (E) an eine Ausgabeeinheit weiterleiten kann,
• eine Ausgabeeinheit (30) welche so ausgebildet ist, dass sie die Klassifikationsdaten (E) der Auswertungseinheit (20) empfangen und dann ausgeben kann.

4. Vorrichtung zum Hammerschlagmonitoring gemäß Anspruch 3 **dadurch gekennzeichnet, dass** dieses wenigstens weiterhin eine Eingabeeinheit (10) umfasst, welche so ausgebildet ist, dass über diese wenigstens ein klinischer Parameter (D2) eingegeben und an eine Auswertungseinheit (20) weitergeleitet kann, und weiterhin
eine Datenschnittstelle (50), welche so ausgebildet ist, dass sie Audio-Messdaten (D1) und/oder den wenigstens einen klinischen Parameter (D2) aus einem externen Datenverarbeitungssystem empfangen und die Klassifikationsdaten (E) an ein externes Datenverarbeitungssystem weiterleiten kann umfasst wobei die wenigstens eine Datenschnittstelle (50) weiterhin den wenigstens einen klinischen Parameter (D2) aus einem externen Datenverarbeitungssystem und die Klassifikationsdaten (E) an ein externes Datenverarbeitungssystem weiterleiten kann,
und wobei die wenigstens eine Auswertungseinheit (20), weiterhin so ausgebildet ist und/oder den wenigstens einen klinischen Parameter (D2) von der Eingabe-einheit (10) oder der Datenschnittstelle (50) empfangen kann aus den vorverarbeiteten Audio-Messdaten (D1*) und dem wenigstens einen klinischen Parameter (D2) Klassifikationsdaten (E) für wenigstens einen Schlag eines Operationshammers auf eine zu implantierenden Prothese durch das künstliche Neuronale Netz ermitteln kann und diese Klassifikationsdaten (E) an eine Ausgabeeinheit weiterleiten kann.

## Claims

1. Method for hammer strike monitoring for monitoring implantations of prostheses in bone by means of a surgical hammer, comprising at least the following steps
a) recording the sound emissions in an operating theatre as audio measurement data (D1) during an implantation and transferring the audio measurement data (D1) to a trained artificial neural network (ANN)
b) pre-processing and segmentation of the audio measurement data (D1) from step a) by the trained artificial neural network (ANN), so that the audio measurement data generated by hammer strike of a surgical hammer on a prosthesis are extracted and pre-processed audio measurement data (D1*) are generated.
c) determining the classification data (E) at least from the pre-processed audio measurement data (D1*) from step b) by means of the trained artificial neural network (ANN), which comprise at least one classification value for at least one stroke of a surgical hammer on a prosthesis,
so that this classification data (E) can be used to evaluate the impact quality, the pre-processed audio measurement data (D1*) being used over the entire course of an operation
d) output of the classification data (E) from step c) for monitoring implantations of prostheses

2. Method according to claim 1, **characterised in that** this further comprises a step a1), which takes place before step c), in which at least one clinical parameter (D2) is received and transferred to the trained artificial neural network (KNN), so that in step c) the determination of the classification data (E), at least from the preprocessed audio measurement data (D1*) and the at least one clinical parameter (D2) is determined by means of the trained artificial neural network (KNN), wherein the classification data (E) comprise at least one classification value for at least one strike of a surgical hammer on a prosthesis

3. Hammer strike monitoring device for carrying out a method according to claim 1, **characterised in that** it comprises at least the following elements:
- a microphone (5) which is designed so that it can generate audio measurement data (D1) from sound emissions and transmit it to an evaluation unit (20),
- an evaluation unit (20) with a trained artificial neural network (ANN), which is designed such that it can receive the audio measurement data (D1) from the microphone (5), preprocess and segment the audio measurement data (D1) and thus generate preprocessed audio measurement data (D1*), and is further designed such that it can generate preprocessed audio measurement data (D1*) by means of the trained artificial neural network (ANN), that it can use the trained artificial neural network (KNN) to determine classification data (E) from the preprocessed audio measurement data (D1*) for at least one impact of a surgical hammer on a prosthesis to be implanted by the artificial neural network and can forward this classification data (E) to an output unit,
- an output unit (30) which is designed such that it can receive the classification data (E) from the evaluation unit (20) and then output it.

4. Device for hammer strike monitoring according to claim 3, **characterised in that** this at least further comprises an input unit (10) which is designed in such a way that at least one clinical parameter (D2) can be input via this and forwarded to an evaluation unit (20), and furthermore a data interface (50) which is designed such that it can receive audio measurement data (D1) and/or the at least one clinical parameter (D2) from an external data processing system and can forward the classification data (E) to an external data processing system
wherein the at least one data interface (50) can further forward the at least one clinical parameter (D2) from an external data processing system and the classification data (E) to an external data processing system,
and wherein the at least one evaluation unit (20) is further configured and/or can receive the at least one clinical parameter (D2) from the input unit (10) or the data interface (50) from the preprocessed audio measurement data (D1*) and the at least one clinical parameter (D2), can determine classification data (E) for at least one strike of a surgical hammer on a prosthesis to be implanted by the artificial neural network and can forward this classification data (E) to an output unit.

## Revendications

1. Procédé de surveillance des coups de marteau pour la surveillance d'implantations de prothèses dans des os au moyen d'un marteau chirurgical, comprenant au moins les étapes suivantes
a) saisie des émissions sonores dans une salle d'opération sous forme de données de mesure audio (D1) pendant une implantation et transfert des données de mesure audio (D1) à un réseau neuronal artificiel (RNA) entraîné
b) prétraitement et segmentation des données de mesure audio (D1) de l'étape a) par le réseau neuronal artificiel entraîné (KNN), de manière à extraire les données de mesure audio générées par les coups de marteau d'un marteau chirurgical sur une prothèse et à générer des données de mesure audio prétraitées (D1*).
c) détermination des données de classification (E) au moins à partir des données de mesure audio prétraitées (D1*) de l'étape b) au moyen du réseau neuronal artificiel (RNA) entraîné, lesquelles comprennent au moins une valeur de classification pour au moins un coup d'un marteau d'opération sur une prothèse,
de sorte qu'une évaluation de la qualité du coup peut être effectuée à partir de ces données de classification (E), les données de mesure audio prétraitées (D1*) étant utilisées sur l'ensemble du déroulement d'une opération.
d) sortie des données de classification (E) de l'étape c) pour la surveillance des implantations de prothèses

2. Procédé selon la revendication 1, **caractérisé en ce qu'**il comprend en outre une étape a1), qui a lieu avant l'étape c), dans laquelle au moins un paramètre clinique (D2) est reçu et transmis au réseau neuronal artificiel entraîné (KNN), de sorte qu'à l'étape c), la détermination des données de classification (E), au moins à partir des données de mesure audio prétraitées (D1*) et de l'au moins un paramètre clinique (D2) au moyen du réseau neuronal artificiel entraîné (KNN), les données de classification (E) comprenant au moins une valeur de classification pour au moins un coup d'un marteau d'opération sur une prothèse

3. Dispositif de surveillance des coups de marteau pour la mise en oeuvre d'un procédé selon la revendication 1, **caractérisé en ce qu'**il comprend au moins les éléments suivants :
- un microphone (5) qui est conçu de telle sorte qu'il peut générer des données de mesure audio (D1) à partir d'émissions sonores et les transmettre à une unité d'évaluation (20),
- une unité d'évaluation (20) avec un réseau neuronal artificiel (RNA) entraîné, qui est conçue de telle sorte qu'elle peut recevoir les données de mesure audio (D1) du microphone (5), prétraiter et segmenter les données de mesure audio (D1) et générer ainsi des données de mesure audio prétraitées (D1*) et est en outre conçue de telle sorte, qu'elle peut déterminer, au moyen du réseau neuronal artificiel (KNN) entraîné, à partir des données de mesure audio (D1*) prétraitées, des données de classification (E) pour au moins un coup d'un marteau d'opération sur une prothèse à implanter par le réseau neuronal artificiel et qu'elle peut transmettre ces données de classification (E) à une unité de sortie,
- une unité de sortie (30) qui est conçue de telle sorte qu'elle peut recevoir les données de classification (E) de l'unité d'évaluation (20) et les délivrer ensuite.

4. Dispositif de surveillance des coups de marteau selon la revendication 3, **caractérisé en ce qu'**il comprend au moins en outre une unité d'entrée (10) qui est conçue de telle sorte qu'au moins un paramètre clinique (D2) peut être entré par l'intermédiaire de celle-ci et transmis à une unité d'évaluation (20), et en outre
une interface de données (50) qui est conçue de telle sorte qu'elle peut recevoir des données de mesure audio (D1) et/ou le au moins un paramètre clinique (D2) d'un système de traitement des données externe et transmettre les données de classification (E) à un système de traitement des données externe
dans lequel l'au moins une interface de données (50) peut en outre transmettre l'au moins un paramètre clinique (D2) d'un système de traitement de données externe et les données de classification (E) à un système de traitement de données externe,
et dans lequel l'au moins une unité d'évaluation (20) est en outre réalisée de cette manière et/ou peut recevoir l'au moins un paramètre clinique (D2) de l'unité d'entrée (10) ou de l'interface de données (50) à partir des données de mesure audio prétraitées (D1*) et de l'au moins un paramètre clinique (D2) et peut déterminer des données de classification (E) pour au moins un coup d'un marteau d'opération sur une prothèse à implanter par le réseau neuronal artificiel et peut transmettre ces données de classification (E) à une unité de sortie.
